# EUROPEAN PATENT SPECIFICATION

(11) **EP 1 054 587 B1**
(45) Date of publication and mention of the grant of the patent: **14.09.2005**
(21) Application number: 98958091.5
(22) Date of filing: 19.11.1998
(51) Int. Cl.: A01C 1/04, A01C 1/06, A01C 1/00

(54) **SOLID MATRIX CONDITIONING OF SEEDS**
FESTES MATRIX ZUR KONDITIONIERUNG VON SAMEN
CONDITIONNEMENT DE SEMENCES SUR UNE MATRICE SOLIDE

(30) Priority: 26.11.1997 US 979074; 26.11.1997 US 979075
(43) Date of publication of application: 29.11.2000
(60) Divisional application: 03013390.4
(73) Proprietor: Kamterter II, L.L.C., Lincoln, NE 68503 (US)
(72) Inventor: Eastin, John Alvin, Lincoln, NE 68503 (US)
(74) Representative: UEXKÜLL & STOLBERG
(86) International application number: PCT/US1998/024733
(87) International publication number: WO 1999/026467

(56) References cited:
- EP-A- 0 686 340
- GB-A- 1 568 174
- US-A- 4 403 445
- US-A- 4 837 970
- US-A- 4 912 874
- US-A- 5 628 144

## Description

This invention rotates to the sorting of damaged seeds from undamaged seeds and particularly the sorting of damaged seeds from undamaged seeds as part of a seed conditioning process.

It is known from United States Patent 5628144, to prime seeds by mixing the seeds, a particulate solid matrix material and a seed conditioning amount of water, for a time and at a temperature sufficient to cause the seeds to imbibe sufficient water to enhance water absorbing characteristics of seed material with the seed hull. The seeds are unchanged in size during this process. Whether the seeds are primed or not, there will be some damaged seeds among the undamaged seeds. The planting of such a mixture of undamaged and damaged seeds is less efficient and wasteful of resources as compared to the planting of only undamaged seeds.

It is also known from United States Patent 4403445, to sort stringless green beans from string beans by soaking the beans in water wherein the placenta of the stringless beans swells making it easier to sort them from string beans that do not have the placenta. This method does not sort physically damaged seeds but only those seeds that have the characteristic of an undesirable string in some beans within a lot of beans and which have a placenta that remains attached to the stringless beans and not to the string beans.

Accordingly, it is the purpose of the invention to provide a novel method of sorting damaged seeds from undamaged seeds especially as an added step to a seed priming function.

This purpose is accomplished by separating the swollen seeds from unswollen seeds by size after the seeds have been water conditioned by adding large amounts of water for a short time to cause swelling of damaged seeds sufficient for separation from undamaged seeds but before harm is done to the undamaged seeds from the water. Advantageously, the seeds are moved in a flow through process while the seeds are immersed in water.

In one embodiment, the seeds are mixed with a particulate solid matrix material and a seed priming amount of water with sufficient aeration, for a time and at a temperature sufficient to cause the seeds to imbibe sufficient water to enhance resultant plant vigor but insufficient to cause seed sprouting.

The sorted undamaged seeds are primed and the primed seeds are planted under conditions not within one of a threshold germination temperature range and a threshold germination moisture range for unprimed seeds of the same species but within at least one of a threshold germination temperature range and a threshold germination moisture range for the primed seed. The matrix material may either be removed before planting the seed or the matrix material may be planted with the seed.

The above noted and other features of the invention will be better understood from the following detailed description when considered with reference to the accompanying drawings in which:
FIG. 1 is a block diagram of a priming system in accordance with an embodiment of the invention;
FIG. 2 is a diagrammatic view of a portion of a mixing system used in the embodiment of FIG. 1;
FIG. 3 is a diagrammatic view of another embodiment of mixing system used in accordance with the embodiment of FIG. 1 instead of using the embodiment of FIG. 2;
FIG. 4 is a diagrammatic view of a conditioning system used in an embodiment of a portion of FIG. 1;
FIG. 5 is a diagrammatic view of a drying system used in accordance with the embodiment of FIG. 1;
FIG. 6 is a diagrammatic view of another embodiment of drying system in accordance with the invention;
FIG. 7 is a diagrammatic view of still another embodiment of drying system used, with the invention; and
FIG. 8 is a diagrammatic view of a separator assembly according to the invention.

To prime seeds, the seeds are incorporated in a solid phase matrix comprising finely divided nonpathogenic, water-holding solids. Preferably, the matrix material, not including the seeds, is in such proportion and distribution with respect to the seeds so that the seed surfaces are sufficiently aerobic to favor aerobic metabolism of the seeds and of beneficial microorganisms, to deter the growth of unfavorable faculative anaerobes or anaerobic microorganisms and to permit proper metabolic changes. In most cases, this is accomplished by using the proper volumetric ratio of seed to matrix material and aeration.

The volumetric ratio of seed to matrix material should be for practical reasons in the range of one volume of seed to 120 of matrix material at one extreme to one of matrix to ten of seed at the other extreme. preferably, a one to one ratio of seed to matrix is generally favorable but the ratio may be lower as in cases where seed exudation and germination inhibitor removal are significant seed priming and treatment objectives and higher such as when microbial inoculation calls for retaining exudates. In cases where the seed to matrix mixture contains a substantially larger volumetric ratio of seed over matrix, proper aeration during priming can be maintained through a combination of mechanical mixing and aeration of the seed plus matrix mass or more aeration.

The matrix material, when containing the water necessary to prime the seeds in question, should be sufficiently friable, nonclumping etc. so that, when desired, it can be separated from the treated seeds after treatment without damage to the seeds. The particle size of the matrix material is not unduly critical as long as surface area is adequate, but for mechanical reasons should be smaller or larger than the seed being treated, usually less than 20 mesh and preferably substantially different. Typically, a material less than about 60 mesh is preferred; for example the Agro-Lig described hereinafter was 90%/wt less than 200 mesh, the soft coal was less than 60 mesh.

One type of matrix is formed of organic solids, for example a carbonaceous, preferably a lignateous solid which has a large equilibrium water potential and preferably has an osmotic potential component which is at least about 90% and preferably greater than 95% of the total water potential, measured as described below. Examples of such material include coal, especially soft coal, lignateous shales such as the leonardite shale, sold as Agro-Lig, and sphagnum moss.

Another type of matrix material is inorganic such as calcined clay mineral, vermiculite and perlite. To achieve a practical cost-effective system using an inorganic particulate matter as the matrix, the bulk density of a matrix of that inorganic particulate matter is preferably above 0.3 grams per cubic centimeter. In this specification, bulk density means the weight in grams of a given volume of particulate material divided by its volumes in cubic centimeters. When using inorganic materials, the water potential is generally determined not primarily by osmotic potential but primarily by matric potential.

In the process of the invention, the seed to be treated, a predetermined amount of solid matrix material and a predetermined amount of water are admixed and the mixture allowed to equilibrate, preferably in a container designed to provide aeration but which reduces evaporative losses, for example, a closed metal container or bag, for a time and at a temperature sufficient to allow the seeds to imbibe water from the matrix and maintain or change a prescribed water content equilibrium temperature and aeration sufficient to enhance resultant plant vigor, i.e., enhance emergence, growth or yield characteristics, but short of that which would cause the seed to sprout. Particularly useful materials for the matrix are coal related materials, calcined clay, diatomaceous earth vermiculite, sawdust, perlite, peat/moss, corn cobs and grain dust.

As in solution priming, the equilibrium amount of water for the system to prime the seed is dependent on the specific seed variety, its state or condition, and the water potential of the solid matrix material. Typically the solid matrix material should have a water potential between about -0.5 to about -0.2 megapascals at equilibrium with the seeds. With control of temperature, this range may be extended to -0.2 to about -3.6. The exact conditions depend on the objectives and species such as destruction of inhibitors or physiological or physical blockages. The seed priming art to some extent is still empirical, and while typical water amounts and media water potentials for given seed types are already generally known from the solution priming art and solid state matrix priming experiments for some seeds, it is frequently best to test a small sample of a new seed over a readily determined range of water potentials and temperatures to determine what conditions of temperature, water potential and time cause appropriate imbibing of water by the seed and resultant pregermination events. After this priming, the seeds may be dried to a resting or dry storage state, with or without the matrix material. Treatment with beneficial microbes or chemical treatment may be before, during or after priming.

In one process of the invention, a known weight of seed is wet with about 25% by weight of water. The total water utilized is typically in the order of about one liter per kilogram of seed but varies with seed size and seed condition. The seed is mixed with the dry, flowable, particulate solid matrix material and water with appropriate chemicals or biologicals added so as to wet seeds and particulate matrix material uniformly. After the predetermined amount of water for priming is admixed with the coated seeds, the mixture is held at a predetermined temperature for a time sufficient to allow the seeds to maintain a desired moisture content equilibrium, usually one to about fourteen days. In other processes, the water, seed and matrix material are mixed in a continuous flow or batching blending system. The ratio of water may also vary substantially from 25 percent of the seed weight.

The seeds that can be treated can be virtually any seed, including most vegetable crops, ornamentals and agronomic crops. Included are cucumber, lettuce, carrot, onion, melons, sweet corn, tomatoes, eggplant, peppers, bean radish, squash, pea, flower seeds, alfalfa and soybean. Several different apparatus can be used for these procedures. One such apparatus is described below.

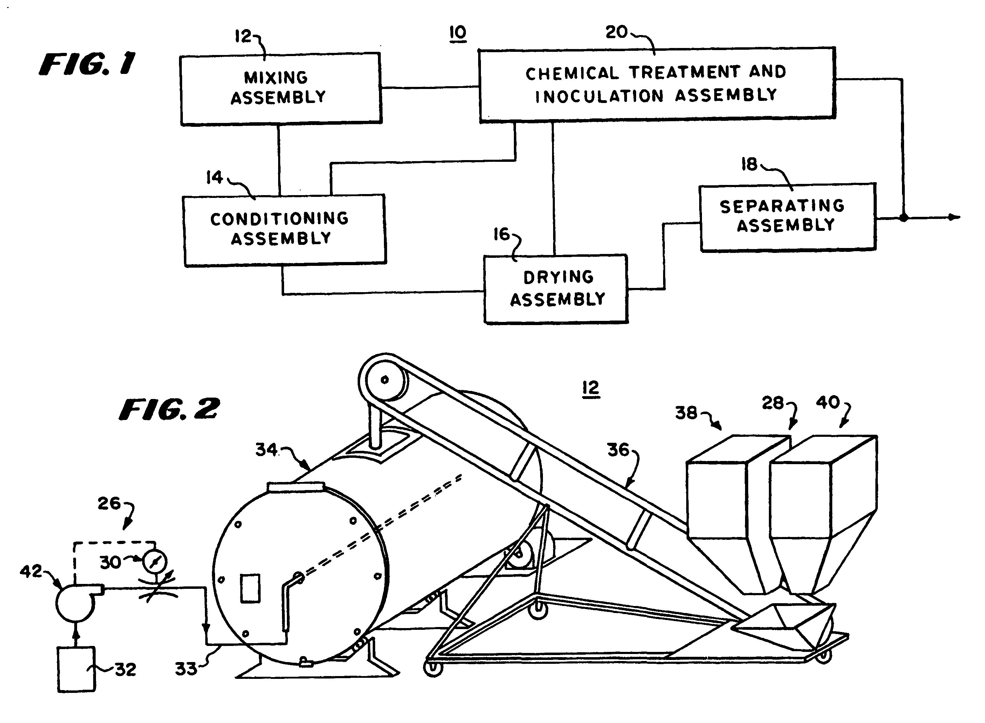

In FIG. 1, there is shown a block diagram of a priming system 10 having a mixing assembly 12, a conditioning assembly 14, a drying assembly 16, a separating assembly 18 and a chemical treatment and inoculation assembly 20. In this system, seeds and a matrix are: (1) mixed together either with no chemical treatment or inoculation of the seeds or with chemical treatment and/or inoculation of the seeds; (2) conditioned; (3) dried back; and (4) in some embodiments, the matrix medium and seeds are separated.

The chemical treatment or inoculation generally takes place in the chemical treatment and inoculation assembly 20 which may communicate with the mixing assembly 12, the conditioning assembly 14, the drying assembly 26 or the separating assembly 18 or none of them. The mixed matrix, seeds and water are utilized for pre-germination metabolism, after which the seeds may be dried back in the drying assembly 16 although from time to time the drying assembly 16 and conditioning assembly 14 are used intermittently for staged conditioning and drying. The drying assembly 16 may communicate with the separating assembly 18 to separate the matrix from the seed in some embodiments although such separation is not always required. The treated seeds may be stored and used later or immediately utilized.

Not all seeds are subject to chemical treatment or inoculation, and thus, the chemical treatment and inoculation assembly 20 is not always used. However, in some embodiments, the seeds may be chemically treated or inoculated with microbes prior to being mixed in the mixing assembly 12 with water and matrix, or in the alternative, such treatment may be applied in the conditioning assembly 14, the drying assembly 16 or later after preconditioning.

In some embodiments, the beginning stages of metabolism occur until the beginning of germination as indicated by the emergence from the seed hull of embryo, at which time inoculation with beneficial microbes takes place. After the inoculation, the seeds may be returned to the conditioning assembly 14 for healing to be restored to a pre-germination stage without full germination and be dried back for later use.

In FIG. 2, there is shown a schematic view of a mixing assembly 12 having as its principal parts a water source 26, a solid matter source 28 and a rotatable mixing tank 34 connected together so that the source of water or water plus additives such as potassium nitrate and the solid matter source 28 such as matrix material and seeds communicate with a rotatable mixing tank 34 which combines the ingredients into the proper mixture for oxidation, temperature control and water potential control. All of these sources may pre-mix the solid matter and moisture and then apply it to the mixing tank or they may be individually applied or combined to pairs and applied or combined with treatment material or the chemical treatment material may be applied to any of the ingredients such as the source of water or the source of matrix or to the seeds alone.

The source of water 26 includes a meter 30, a source of water 32, and in some embodiments a pump 42 connected together so that a metered amount of moisture may be applied to a mixing mechanism, which in the embodiment of FIG. 2, is a rotatable tank. It is adapted to receive the water and solid matter and then to be rotated for thorough mixing. Of course, other embodiments may be utilized such as a tank with internal stirring means rather than a rotating tank and/or connections with rotatable universal joints that rotate with the rank rather than connectors at the center of rotation.

The solid matter source 28 includes a seed and solid matrix conveyor 36, a source of seeds 38, and a source of matrix material such as clay 40. These ingredients may be premixed or moved one-by-one into the tank 34 by an auger or forced air flow or gravity or any other means.

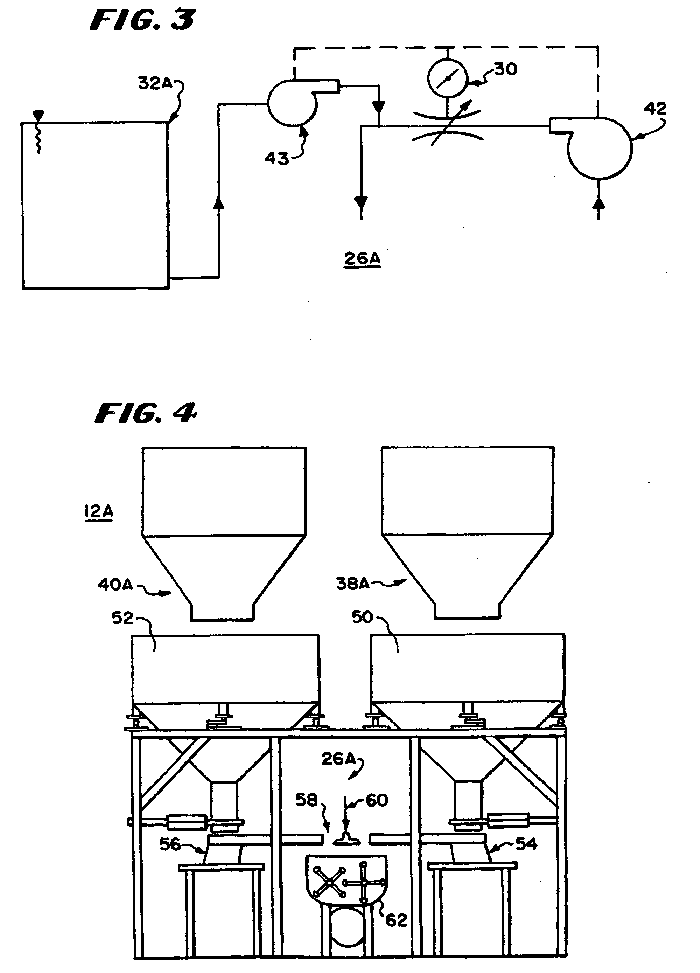

In FIG. 3, there is shown an embodiment of water source 26A having a tank of water 32A which contains water and potassium nitrate. The tank communicates with a pump 42 and a meter 30 in a conduit 31 for supplying a mixture of liquid ingredients which may include additives such as potassium nitrate to condition for light sensitivity or chemicals beneficial to seed treatment or being antifungal or antimicrobial or with beneficial microbials for inoculation and in some embodiments, nutrients therefore.

With this arrangement, a carefully metered amount of water may be supplied to provide the correct matrix potential. In some applications, a small amount of water is supplied and then the supply is disconnected from the tank which is rotated for mixing and then further water is supplied. The intermittent stationary portions of the tank may be utilized to also add more dry matter if desirable so that the mixing together may proceed with stepped amounts of dry matter and water.

In FIG. 4, there is shown a schematic diagram of another embodiment of mixing assembly 12A having a source of seeds 38A, a source of dry matter 40A, the source of water and liquid additives 26A, a mixing section 58, and a mixture agitator and transporter 62. The source of seeds 38A and the source of dry matter 40A each include a different one of the hoppers 50 and 52 respectively for containing bulk seed and dry matter such as matrix. At the bottom end of each, there is a corresponding one of the seed conveyors 54 and conveyor 56 which are aligned directly in the line of gravity underneath the seed and matrix hoppers 50 and 52 respectively and are drivable at controlled speeds independently to transport from the hoppers controlled amounts of material for mixing in the mixing station 58.

The bottoms of the hoppers are adjustable and the speed of the containers are adjustable so that the ratio of mixing may be proportional in accordance with the type of seed and the type of matrix material. Similarly, the water supply 26A supplies water at a controlled rate through a conduit 60 into the mixing station 58 so that controlled amounts of matrix material, seed and water or water solutions are mixed together at the mixing station 58 in a continuous process for thorough mixing with the station 58 receiving at any one time between 1 percent and 60 percent of the total amount a batch to be processed during batch processing, or if continuous processing is used, a volume of properly proportioned materials which is adapted to fit into a flow stream no faster than the slowest processing of that stream. The proportional mixtures are mixed at the station 58 and conveyed by the mixer, agitator and transporting system 62 which moves the mixture along by a series of panels to an outlet where it may be conveyed to the seed conditioner. The seed conditioner may accumulate it batch by batch for processing or the seeds may be conditioned in a continuous process.

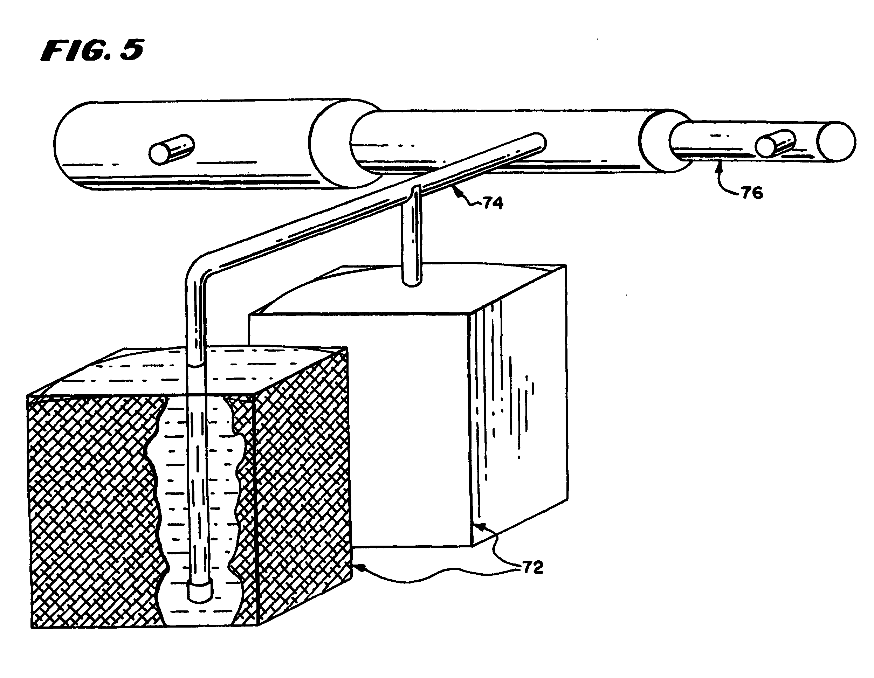

In FIG. 5, there is shown a conditioner 14 which includes a means for controlling the temperature, moisture and oxygen supply of the matrix containing seeds to promote pre-germination conditioning. In the embodiment of FIG. 5, all of these functions are performed in a unitary manner by a porous fabric bag 72 which contains the matrix and seeds, a suction manifold 74 positioned for even moisture and temperature drawing of air through the fabric bag 72 and matrix to a vacuum manifold 76 and a temperature and moisture controlled room for this processing. For very small amounts, the air drawing supply 74 is unnecessary since there will be adequate natural transfer of heat and oxygen through the matrix material for the desired purposes.

The fabric bag 72 has a open end for supplying the mixture to the bag and an openable bottom end for supplying conditioned seeds to a transport conveyor 80 after a conditioning stage. In practice, the seeds may be conditioned in the fabric bag 72 and then transported for drying back through a 10 percent stage of drying and then returned to the bag for further conditioning. Moreover, the conditioning may extend until the beginning of emergence and inoculated and dried and conditioned again prior to emergence.

To draw air through the fabric bag 72, a centrally located vacuum pipe 74 extends downwardly. It is designed so that the larger surface area on the outside of the fabric bag 72 pulls cool dry air through the wider area, and as it picks up some heat and moisture, the rate of flow becomes greater since it is moving inwardly through spheres of smaller area to maintain uniformity of temperature through the bag and relative uniformity removal of moisture. In the preferred embodiment, the bags generally contain between 20 pounds and 1,000 pounds of mixture, contained in a room that is between 10 degrees Centigrade and 15 degrees Centigrade with air being pulled from a manifold under a vacuum pressure of a negative 10 pascals through an outer surface area of 75 square feet to an inner perforated conduit having a diameter of between 1 inch and 15 inches and open pores and ends for drawing air having a combined area of 75 square centimeters.

After a conditioning stage, the fabric bags 72 may be physically moved to driers and dumped into the driers in a manner shown herein or the bottoms may be opened and conveyor mechanisms 80 may convey the mixture to driers or to a drying stage.

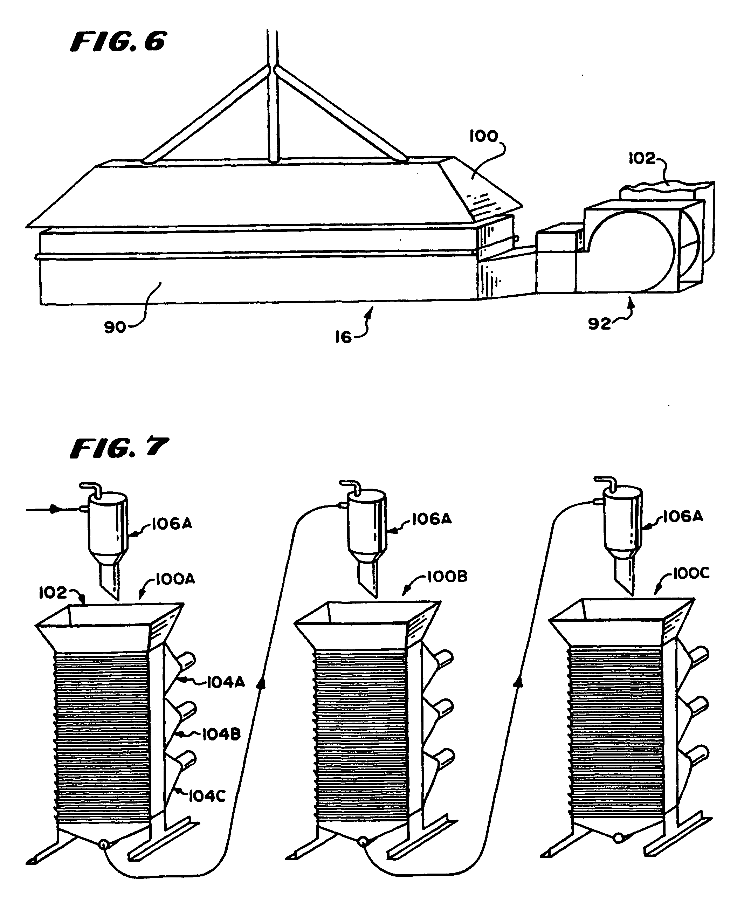

In FIG. 6, there is shown one embodiment of drying assembly 16 having a bin assembly 90, a blower assembly 92, and in some embodiments a cover assembly 100. A source of air pressure 102 applies air through blowers or the like to the blower assembly beneath the bin assembly 90. The bin assembly 90 has a fabric bottom through which the air pressure blows to cause turbulence in the matrix material spread across the bin assembly 90 for drying thereof by air flow. A cover may be placed over the bin assembly 90 to create slight negative pressure if desired for dust control and moisture may be extracted by recirculation of the air through desiccators in the cover.

In one embodiment, air pressure in the manifold beneath the bin assembly 90 of a positive 50 pounds per square inch is applied through a 60 gauge fabric for drying matrix material applied in a layer between 4 inches and 3 feet deep and having a pressure on the fabric of between 6 ounces and 15 pounds per square inch.

In FIG. 7, there is shown another embodiment of drying assembly having a plurality of drying towers 100A, 100B, and 100C each of which receives a mixture of matrix and seed for drying with the seed being applied to the top of the tower 100A through a conveyor 106A which may be an auger or an air blower or the like for filling the tower 100A. The tower 100A is emptied into the tower 100B through a similar conveyor 106A and so on down the line until the drying operation is completed, after which the mixture may be moved to a separator for sifting and separating or may be stored for use in a planter with the matrix material and seed combined or moved to a location for chemical treatment or biological inoculation. Each of the towers is identical and so only the tower 100C will be described.

To provide a stage of drying, the drying tower 100C contains an elevated bin 102 having a plurality of stages of air flow conduits 104A, 104B and 104C. Any number of such stages may be supplied and they include within them a blower for blowing air crosswise through the tower where it is received by the next section for blowing crosswise again through the tower from the opposite side forwardly so that the air blows across the tower through several stages, three being shown for illustration in FIG. 7. A desiccant or other dryer may be utilized at each stage to remove moisture from the air as it dries the mixing combination.

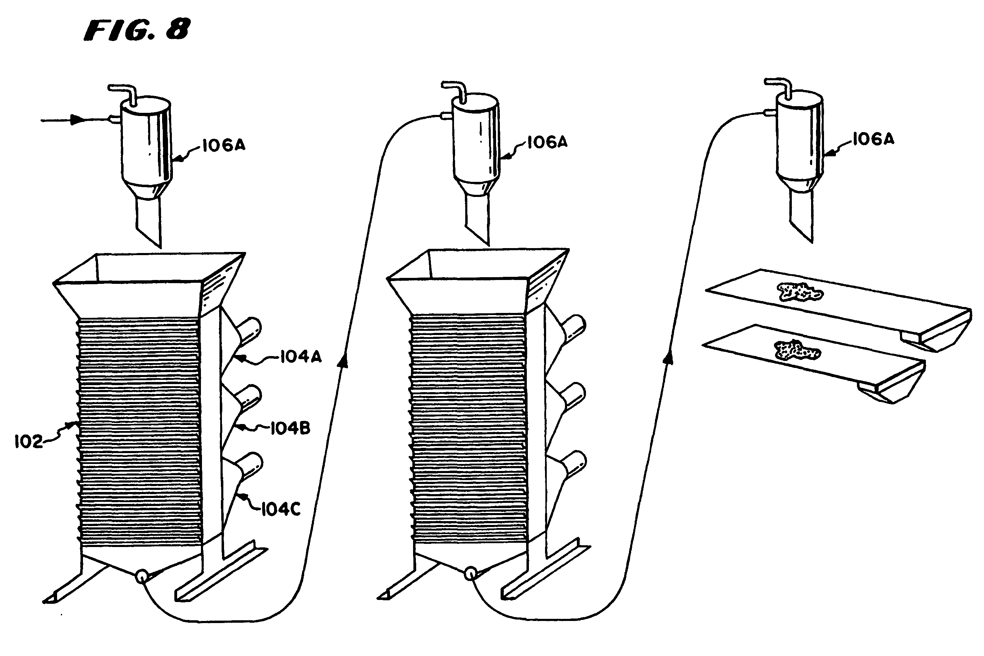

In FIG. 8, there is shown one embodiment of separator for separating the matrix material from the seed. This embodiment includes commerial sifters with gauge shifting designed to hold the seed but permit the matrix to fall through or to hold the matrix and permit the seed to fall through or to sort the seed and matrix at two different slides or conveyors for convenient separation.

With this arrangement, seeds are primed using a solid phase matrix, rather than using aqueous solutions. The matrix comprises finely divided non-plant-pathogenic, water-holding solids. Preferably, the matrix material, not including the seeds, is in such proportion and distribution with respect to the seeds so that the seed surfaces are sufficiently aerobic to: (1) favor aerobic metabolism of the seed and of beneficial microorganisms; (2) deter the growth of unfavorable faculative anaerobes or anaerobic microorganisms; and (3) to permit proper metabolitic changes. In most cases, this is accomplished by using the proper volumetric ratio of seed to matrix material and aeration.

A slight modification of this equipment permits the screening out of bad seeds. In this method, higher gravity or larger seed particles are eliminated by screens or agitating until the larger seed particles are removed. In this specification "seed particles" means the seed and material adhered to the seed by sticking to seed exudate. This process is a result of noticing that injured seeds exude more than healthy seeds and thus more matrix material or the like stick to them.

Another method of sorting defective seeds from seeds that are to be planted using sizing or screening techniques takes advantage of controlled moisture conditioning that is part of the solid matrix priming technique and may use the same equipment. Seeds which have been damaged by cracking may be efficiently separated from seeds that have not been so damaged during a water conditioning process on a mass production scale taking advantage of certain newly discovered or newly applied principles. In the method, if there are differences in the sizes of the seeds, such as for example 10 percent or greater differences, the seeds are initially sized, such as for example, into batches of small seeds, batches of medium seeds and batches of large seeds or other divisions that enable the later distinction between enlarged seeds and seeds not enlarged. This is necessary to permit a separation of good seeds from damaged seeds based on percentages of increase in the size of damaged seeds without confusing an enlarged small damaged seed that is to be separated out from a good naturally large seed during the screening process.

After sorting into batches by size if this is necessary, the seeds are moisture conditioned so that the moisture within the seed is brought to the proper moisture, such as for example, soybean seeds will frequently be at eight percent moisture when unconditioned. For sorting the damaged seeds, the moisture level is adjusted to the desirable moisture to increase plant vigor, such as for example in the case of soybean seeds, to a 12 percent moisture level. At this moisture level, the internal seed material may rapidly receive water and expand but the outer shell serves as a barrier. When the moisture level has been so conditioned, the outer shell is leathery and strong so that the seeds may be easily moved about and sorted in sorting equipment through different screen sizes.

At this point in time, which will take different amounts of time for different seeds, such as for example, eight to ten hours with soybean seeds, the seeds are ready for sorting. They are sorted by adding a large quantity of water such as by immersing the entire batch into water for a few minutes, such as for example three minutes for soybean seeds. At the end of that three minutes, the damaged seeds will have swelled sufficiently in comparison to the undamaged seeds to permit sorting in spite of variations of seed sizes within the batch. For example, in the case of soybean seeds, the amount of expansion of damaged seeds may be as high as 25 to 40 thousandths of an inch or more than ten percent greater in enlargement than the undamaged seeds. This is probably because cracks in the damaged seeds permit water to pass readily through the shell into the inside seed material which, because of its conditioning, is in an optimum stage to receive water quickly and swell whereas the undamaged seeds provide a barrier in its intact outer shell although the interior material is equally ready to receive water.

With this mechanism, in a few minutes, such as two to five minutes, the seeds may be put into a condition in which the undamaged seeds may be sorted readily from the damaged seeds. The time of emersion in water is sufficiently low so that the seeds are not damaged by emersion which they would be with a longer period of time. By this method, combining conditioning of the seeds to the appropriate moisture level with a short emersion in a large amount of water, seeds may be separated on a production scale to reduce the number of seeds that will not germinate in the field. One such method is to move the seeds to a screening apparatus while the seeds are substantially immersed in water.

The solid matrix priming compositions described above can also advantageously contain: (1) prescribed amounts of known seed fungicides such as thiram, captan, metalaxyl, pentachloronitrobenzene, and fenaminosulf, so that the finished primed seed is coated with or absorbs the desired amount of fungicide, such as is known to those skilled in the art; (2) microorganisms useful to the crop such as those useful in crop protection, stimulation or establishment, and for such purposes, some significant bacteria are strains of: Bacillus enterobacter, Pseudomonas, Rhizobia and Serratia species and some significant fungi are strains of Trichoderma, Gliocladium and Laetisaria species; (3) pesticides such as fungicides or bactericides included prior to, during or after the solid matrix priming; and (4) growth regulators such as potassium nitrate, gibberellic acid, DCPTA, ethephon.

In this specification, "solid matrix priming" is considered the process whereby seeds are intimately mixed with particulate solid phase media in the presence of sufficient water to realize a moisture content in the seed which allows for germination processes to occur but which prevents radicle emergence. During priming and especially during solid state priming, the addition of chemical additives and microorganisms is especially beneficial. In this specification, the language "added beneficial microorganisms" means a beneficial microorganism that is added to the composition at a level in excess of that naturally occurring on the seed being primed.

In this specification: (1) threshold germination temperature range means that range of temperatures for a certain species within which seeds of that species will germinate at a predetermined moisture level and with adequate oxygen; and (2) threshold germination moisture range means that range of moistures for a certain species within which seeds of the species will germinate at a given temperature and with adequate oxygen.

In addition to the above ranges, the limiting points of moisture and temperature in this specification are defined as follows: (1) threshold germination temperature base means that temperature below which a seed for a given species will not germinate even though the oxygen and moisture levels are acceptable; (2) threshold germination critical maximum temperature means that temperature above which a seed of a given species will not germinate even though there is an acceptable moisture level and adequate oxygen; (3) threshold germination moisture base means that moisture level below which a seed of a given species will not germinate even though the temperature and oxygen are appropriate; and (4) threshold germination moisture critical maximum level means that moisture level in soil above which a seed of a given species will not germinate even though the temperature and oxygen are adequate.

Other convenient terms are defined as follows: (1) emergence rate index means the sum of the emerged seeds, n, on each day multiplied by a quantity for that day, quantity for that day being equal to the total number, c, of days in the assay minus the count, n, of seedlings on that day; and (2) synchronization factor means the emergence rate index that occurs in the period that is one-quarter of the time into the total assay period. The assay period is a normal field crop emergence period. The emergence rate index and synchronization factor can be calculated using seeds germinated instead of seedlings emerged to arrive at a similar number. This may be called a germination rate index.

The above values are determined empirically for a given seed and variety and may be used in planting.

A sufficient amount of matrix or a matrix of material sufficiently adsorbent or absorbent to remove enough inhibitors to prevent delay of more than fifteen percent of the time duration of activation prior to a control time of germination in which the inhibitor is substantially completely removed by an appropriate washing technique is required. The time of germination in this specification is that the visible radicle protrusion can be observed and the activation period occurs when membranes become differentially permeable and conversion occurs from substantially passive solute transport in and from the seed. The amount and type of matrix is also selected to either retain or permanently remove exudates so that: (1) in the case of removal, it is not deleterious such as for example, to encourage pathogen growth; or (2) in the case of retention, to enable growth of innoculated beneficial microbes on seeds.

While a preferred embodiment of the invention has been described with some particularity, many modifications and variations in the preferred embodiment may be made without deviating from the invention. Accordingly, within the scope of the appended claims, the invention may be practiced other than as specifically described.

## Claims

1. A method of sorting damaged seeds from undamaged seeds which comprises the steps of mixing seeds, a particulate solid matrix material and a seed conditioning amount of water, for a time and at a temperature sufficient to cause the seeds to imbibe sufficient water to enhance water absorbing characteristics of seed material within the seed hull **characterized by** separating the swollen seeds from unswollen seeds by size after the seeds have been water conditioned by adding large amounts of water for a short time to cause swelling of damaged seeds sufficient for separation from undamaged seeds but before harm is done to the undamaged seeds from the water.

2. A method according to claim 1 **characterized by** the step of moving the seeds in a flow through process while the seeds are immersed in water.

3. A method according to either of claims 1 or 2 **characterized in that** the seeds are mixed with a particulate solid matrix material and a seed priming amount of water with sufficient aeration, for a time and at a temperature sufficient to cause the seeds to imbibe sufficient water to enhance resultant plant vigor but insufficient to cause seed sprouting.

4. A method according to any of claims 1-3 **characterized in that** the sorted undamaged seeds are primed and the primed seeds are planted under conditions not within one of a threshold germination temperature range and a threshold germination moisture range for unprimed seeds of the same species but within at least one of a threshold germination temperature range and a threshold germination moisture range for the primed seed.

5. A method according to claim 3 **characterized in that** the matrix material is removed before planting the seed.

6. A method according to claim 3 **characterized in that** the matrix material is planted with the seed.

## Patentansprüche

1. Verfahren zum Abtrennen von beschädigten Samenkörnern von unbeschädigten Samenkörnern, welches die Schritte umfasst: Mischen der Samenkörner mit einem Feststoffteilchen-Matrixmaterial und einer Samenkörner-Behandlungsmenge von Wasser über eine Zeitspanne und eine Temperatur, die ausreichen, dass die Samenkörner hinreichend viel Wasser aufnehmen, um die Wasserabsorptionseigenschaften des Samenmaterials innerhalb der Samenschale zu vergrößern, **dadurch gekennzeichnet, dass** die aufgequollenen Samenkörner von den unaufgequollenen Samenkörnern nach der Größe getrennt werden, nachdem die Samenkörner mit Wasser behandelt worden sind, indem große Mengen von Wasser über eine kurze Zeitspanne zugesetzt wurden, um ein Aufquellen der beschädigten Samenkörner zu bewirken, das zur Abtrennung von unbeschädigten Samenkörnern ausreicht, und zwar ehe die unbeschädigten Samenkörner durch Wasser geschädigt werden.

2. Verfahren nach Anspruch 1, **gekennzeichnet durch** den Schritt, dass die Samenkörner in einem Strom **durch** den Prozess bewegt werden, während die Samenkörner in Wasser eingetaucht sind.

3. Verfahren nach Anspruch 1 oder 2, **dadurch gekennzeichnet, dass** die Samenkörner mit einem Feststoffteilchen-Matrixmaterial und einer Samenkörner-Behandlungsmenge von Wasser mit hinreichender Luftzufuhr vermischt werden, über eine Zeitspanne und bei einer Temperatur, die ausreichen, dass die Samenkörner genug Wasser aufnehmen, um die damit einhergehende Pflanzenkraft zu verstärken, die aber nicht ausreicht, dass die Samenkörner keimen.

4. Verfahren nach einem der Ansprüche 1 bis 3, **dadurch gekennzeichnet, dass** die abgetrennten unbeschädigten Samenkörner vorbehandelt werden und die vorbehandelten Samenkörner unter Bedingungen gepflanzt werden, die nicht innerhalb eines Keimschwellwert-Temperaturbereichs oder eines Keimschwellwert-Feuchtigkeitsbereichs für unbehandelte Samenkörner der gleichen Samenart liegt, aber innerhalb von mindestens einem Keimschwellwert-Temperaturbereich oder einem Keimschwellwert-Feuchtigkeitsbereich für die vorbehandelten Samenkörner liegt.

5. Verfahren nach Anspruch 3, **dadurch gekennzeichnet, dass** das Matrixmaterial vor dem Pflanzen der Samenkörner entfernt wird.

6. Verfahren nach Anspruch 3, **dadurch gekennzeichnet, dass** das Matrixmaterial zusammen mit den Samenkörnern gepflanzt wird.

## Revendications

1. Procédé de tri de semences abîmées à partir de semences non abîmées, qui comprend les étapes consistant à mélanger les semences, un matériau de matrice solide particulier et une quantité d'eau de conditionnement de semences, pendant une durée et à une température suffisantes pour amener les semences à s'imbiber suffisamment d'eau pour améliorer les caractéristiques d'absorption d'eau du matériel de semences à l'intérieur de l'enveloppe des semences **caractérisé par** la séparation des semences gonflées des semences non gonflées en fonction de la taille après que les semences ont été conditionnées par l'eau en ajoutant de grandes quantités d'eau pendant une courte durée pour induire un gonflement des semences abîmées suffisant pour la séparation à partir des semences non abîmées, mais avant qu'un dommage ne soit fait aux semences non abîmées par l'eau.

2. Procédé selon la revendication 1, **caractérisé par** l'étape consistant à déplacer les semences dans un procédé à écoulement continu tandis que les semences sont immergées dans l'eau.

3. Procédé selon l'une ou l'autre des revendications 1 ou 2, **caractérisé en ce que** les semences sont mélangées avec un matériau de matrice solide particulier et une quantité d'eau pour la prégermination des semences avec une aération suffisante, pendant une durée et à une température suffisantes pour amener les semences à s'imbiber suffisamment d'eau pour améliorer la vigueur des plantes résultantes, mais insuffisantes pour induire la germination des semences.

4. Procédé selon l'une quelconque des revendications 1 à 3, **caractérisé en ce que** les semences non abîmées triées sont prégermées et **en ce que** les semences prégermées sont plantées dans des conditions n'étant pas une parmi une plage de températures de germination seuil et une plage d'humidité de germination seuil pour les semences non prégermées de la même espèce, mais au moins une parmi une plage de températures de germination seuil et une plage d'humidité de germination seuil pour la semence prégermée.

5. Procédé selon la revendication 3, **caractérisé en ce que** le matériau de matrice est retiré avant de planter la semence.

6. Procédé selon la revendication 3, **caractérisé en ce que en ce que** le matériau de matrice est planté avec la semence.
